Europäisches Patentamt

European Patent Office

Office européen des brevets

(19)

(11) Numéro de publication: **0 234 973**
**A1**

(12) ## DEMANDE DE BREVET EUROPEEN

(21) Numéro de dépôt: **87400087.0**

(22) Date de dépôt: **15.01.87**

(51) Int. Cl.³: **C 12 N 5/00**
C 12 Q 1/04, C 12 Q 1/70
G 01 N 33/569, C 12 N 7/00
//G01N33/543

(30) Priorité: **15.01.86 FR 8600515**

(43) Date de publication de la demande:
**02.09.87 Bulletin 87/36**

(84) Etats contractants désignés:
**ES GR**

(71) Demandeur: **INSTITUT PASTEUR**
**25-28, rue du Docteur Roux**
**F-75724 Paris Cédex 15(FR)**

(72) Inventeur: **Chermann, Jean-Claude**
**2 Clos d'Ergal**
**F-78310 Elancourt(FR)**

(72) Inventeur: **Nugeyre, Marie Thérèse**
**6 rue Dombasle**
**F-75015 Paris(FR)**

(72) Inventeur: **Hazan, uriel**
**127 bld Saint-Michel**
**F-75005 Paris(FR)**

(72) Inventeur: **Barre-Sinoussi, Françoise**
**104 le Capricorne 50 rue d'Erevan**
**F-92120 Issy Les Moulineaux(FR)**

(72) Inventeur: **Achour, Ammar**
**6 rue Manet**
**F-92390 Villeneuve La Garenne(FR)**

(74) Mandataire: **Gutmann, Ernest et al,**
**S.C. Ernest Gutmann - Yves Plasseraud 67, boulevard**
**Haussmann**
**F-75008 Paris(FR)**

(54) **Population de lymphocytes T4 spécifiques du virus LAV, leur procédé d'obtention et leur application à la production de ce virus.**

(57) Population de cellules portant le phénotype T4 des lymphocytes T4, caractérisée en ce qu'elle est constituée essentiellement par des lymphocytes T4X possédant un site de fixation spécifique du virus LAV et en ce qu'elle est exempte de lymphocytes T4Y et ne possédant apparemment pas de récepteur pour ce virus. L'invention concerne aussi un procédé d'obtention de ces cellules T4X par séparation de ces dernières du reste des cellules T4Y. En outre l'invention concerne un diagnostic *in vitro* permettant la discrimination entre porteurs asymptomatiques d'anticorps anti-LAV et personnes présentant des syndromes de lymphadénopathies (SLA) ou atteintes de para-SIDA ou de SIDA et, en cas de réponse positive, le procédé de l'invention permet le suivi de l'évolution de l'infection produite par le virus LAV. Enfin les populations de lymphocytes T4X et plus particulièrement celles appartenant à des lignées permanentes, sont applicables à la production du virus LAV.

EP 0 234 973 A1

Croydon Printing Company Ltd.

Population de lymphocytes T4 spécifiques du virus LAV, leur procédé d'obtention et leur application à la production de ce virus.

L'invention concerne des populations de lymphocytes T4 spécifiques du virus LAV, des procédés d'obtention de ces populations et leur utilisation pour la production de ce virus.

L'invention concerne également une méthode de diagnostic in vitro permettant l'étude de l'évolution de la maladie due à l'infection par le virus LAV et/ou la discrimination entre porteurs asymptomatiques d'anticorps anti-LAV et des personnes présentant des syndromes de lymphadénopaties (SLA) ou atteintes de para-SIDA ou de SIDA proprement dit, c'est-à-dire la maladie connue sous le nom complet de "syndrome d'immunodéficience acquise".

Pour la commodité du langage, il sera fait référence dans ce qui suit aux cellules T4, étant entendu que la désignation T4 s'étend à toutes les cellules portant le phénotype T4. D'une façon générale, l'expression "cellules T4" sera considérée dans le cadre de la présente demande comme s'étendant aussi bien aux lymphocytes humains non permanents, qu'aux cellules appartenant à des lignées permanentes portant le susdit phénotype T4, telles que des lignées connues sous les désignations CEM, H9, HUT, MOLT, etc..

Le virus LAV, agent étiologique des susdites affections, et divers variants de ce virus ont été complètement identifiés dans la demande de brevet européen déposée le 14 septembre 1984 au nom de l'INSTITUT PASTEUR et sous le titre "Antigènes, moyens et méthode pour le diagnostic de lymphadénopathie et du syndrome d'immunodépression acquise", sous la priorité de la demande de brevet britannique n° 83 24800 déposée le 15 septembre 1983. A titre d'exemple, on peut citer les souches de virus LAV déposées à la Collection Nationale des Cultures de Micro-

2

organismes de l'Institut Pasteur de Paris sous les numéros I-232, I-240, I-241 ou I-502. On sait par ailleurs que des virus similaires ont été isolés aux Etats-Unis d'Amérique. Ils ont été dénommés ARV (Jay LEVY et al., Science, 1984, 225, p. 840-884) et HTLV-III (Science, 1984, vol. 224, 497-500). Quoi qu'il en soit, il sera fait référence dans ce qui suit au virus LAV, également dénommé HIV (abréviation anglaise de "Human Immunodeficiency Virus" (virus d'immunodéficience humaine)) selon les règles de nomenclature récemment adoptées pour désigner des virus LAV et tous les virus doués de propriétés équivalentes.

Il avait déjà été reconnu que les lymphocytes T4 (aussi connus sous la désignation "cellules 3Leu") formaient la principale cible du virus. Ces lymphocytes sont reconnaissables par des anticorps monoclonaux, par exemple ceux commercialisés par la firme Ortho Pharmaceutical Corporation sous la désignation OKT4. Ces anticorps monoclonaux ont été décrits plus particulièrement dans la demande de brevet européen n° 18.794 déposée le 25 avril 1980. Il a également été établi que ces anticorps monoclonaux reconnaissaient plus particulièrement une protéine de poids moléculaire de 62.000 daltons (C. Terhorst et coll. Science (1980), 209, 520-521). Cette protéine a été dénommée "antigène T4" par les susdits auteurs. Elle sera ci-après encore désignée comme "protéine T4". Dans ce qui suit les anticorps monoclonaux spécifiques de cette protéine seront appelés "anticorps anti-T4".

Cette propriété de reconnaissance spécifique des lymphocytes T4 par le virus LAV a été utilisée pour la mise au point d'un procédé et de compositions à base d'anticorps anti-T4, visant à bloquer la pénétration des virus LAV dans les lymphocytes T, donc le développement du processus infectieux (demande de brevet français n° 84 14172 déposée le 14 septembre 1984 au nom de l'INSTITUT PASTEUR).

3

On sait, d'autre part, que le rapport du nombre des lymphocytes T4 au nombre des lymphocytes T8 (qui ne sont affectés qu'à un moindre degré par le virus LAV) tend à diminuer chez certains patients atteints du para-SIDA (lymphadénopathies, etc.) et chez les malades atteints du SIDA. Au dernier stade de la maladie, les lymphocytes T4 ont presque disparu du sang circulant. On peut faire l'hypothèse qu'il s'ensuit une réduction, sinon une disparition des autres fonctions des lymphocytes T4, notamment celles liées à la production par eux d'un médiateur soluble ou lymphokine, l'interleukine-2 (IL-2). Il en résulte alors une perturbation des fonctions immunoprotectrices attribuables aux autres lymphocytes, notamment des lymphocytes T8 cytotoxiques, et de leur capacité à se multiplier.

On a rappelé dans ce qui précède un certain nombre de phénomènes qui peuvent être observés, au niveau des lymphocytes, chez des personnes déjà sujettes à un processus infectieux. Or l'expérience montre que ces malades ne constituent vraisemblablement qu'une proportion minoritaire de l'ensemble des personnes qui ont été en contact avec le virus LAV. Il est en effet bien connu que de nombreuses personnes porteuses d'anticorps anti-LAV, ne présentent heureusement pas les signes cliniques des SLA ou du SIDA, et sont apparemment en bonne santé. Mais il est difficile de distinguer parmi ces personnes celles qui vont rester en bonne santé et celles chez lesquelles la maladie évolue. En effet des rapports lymphocytes T4/lymphocytes T8 qui peuvent conserver l'apparence de la normale chez des personnes déjà atteintes, ne permettent pas la discrimination entre personnes porteuses d'anticorps anti-LAV réellement en bonne santé et personnes entrant dans la phase précoce de la maladie. On conçoit que ces observations soient génératrices d'angoisse chez les premières et rendent difficiles le suivi de l'évolution de la maladie chez les secondes.

Un des buts de la présente invention est de fournir les moyens qui permettent cette discrimination entre personnes porteuses d'anticorps anti-LAV, mais en fait toujours encore asymptomatiques et personnes déjà malades.

En effet, il a déjà été montré que seulement une certaine proportion des lymphocytes T4 est infectable par le virus LAV (Science, 1984, 225, p. 59-63, Klatzmann et al). Mais jusqu'à ce jour il n'était pas établi que les lymphocytes T4 non infectés par un HIV (LAV) dans les essais du type de ceux qui avaient été réalisés par KLAZMANN et al ou d'autres chercheurs présentaient des éléments de structure, à tout le moins des phénotypes permettant de les distinguer de ceux qui avaient été infectés.

L'invention repose sur la découverte qu'en fait les lymphocytes T4 susceptibles d'être infectés par un HIV avaient effectivement des caractéristiques distinctes des lymphocytes T4 qui ne l'étaient pas. Celles des cellules qui sont infectables seront appelées cellules T4X dans ce qui suit, alors que les cellules également porteuses du phénotype T4, mais qui ne sont pas infectables et même ne sont pas reconnues par le virus susdit, seront appelées cellules T4Y. L'invention a en outre permis de montrer que les cellules T4X et les cellules T4Y semblent se différencier au niveau même du récepteur pour le virus. En effet, comme il sera vu dans la suite de cet exposé, les cellules T4X, une fois séparées des cellules T4Y, forment des populations infectables "à 100 %" par le virus.

Le procédé selon l'invention de séparation des cellules T4X et T4Y qui met à profit cette découverte est caractérisé en ce qu'il comprend une étape d'incubation d'une population initiale de cellules T4 en présence de virus LAV inactivé, qui s'est avéré pouvoir former un complexe avec les cellules T4X. Une seconde étape du procédé

5

consiste à séparer les cellules T4X sur lesquelles le virus inactivé est fixé, des cellules T4Y qui se sont avérées ne pas former de complexe avec le virus inactivé. De préférence, si l'on veut récupérer des cellules T4X infectables par le virus, une étape supplémentaire d'incubation en l'absence de virus inactivé est nécessaire.

En effet le procédé selon l'invention met à profit deux propriétés supplémentaires des lymphocytes T4X. Ils sont reconnaissables aussi bien par le virus LAV virulent que par les formes inactivées de ce virus. En outre, lorsque les cellules T4X, sur lesquelles avait au préalable été fixé le virus LAV inactivé (notamment par un virus inactivé à la chaleur, par exemple par traitement à une température de 56°C ± 3°C), sont maintenues en incubation en l'absence de virus, une internalisation semble se produire, puisqu'au bout d'un certain temps, les cellules T4X retrouvent leur aptitude à fixer, de nouveau, un virus inactivé ou virulent.

Ces diverses constatations sont à l'origine de plusieurs types d'applications possibles. Un premier type d'application s'intéresse au diagnostic in vitro de l'absence ou au contraire de la présence de l'infection, en particulier lorsque celle-ci ne s'est pas manifestée par des symptômes cliniques apparents. Dans ce dernier cas, l'invention a encore pour but de permettre le suivi de l'évolution de l'infection en cours. Un second type d'application s'intéresse à l'obtention de populations de cellules T4X sensiblement homogènes, utilisables notamment pour la production de quantités importantes de virus ou comme culture celluélaire - test particulièrement sensible pour l'étude de la cytopathogénicité - de nouveaux isolats de HIV ou, pour le cas où cette cytopathogénicité a été reconnue, pour l'étude de la capacité de substances présumées avoir un effet antiviral d'inhiber la capacité du virus infectieux à infecter ces cultures cellulaires-

6

tests. Elles permettent également - cas dans lequel on peut se trouver ramener à des essais de diagnostic _in vitro_ - l'étude du caractère infectieux de compositions contenant des antigènes de HIV (notamment de prélèvements biologiques, par exemple de sérums humains) par mise en contact de ces prélèvements avec ces cultures cellulaires-tests et la détection, après incubation du mélange formé pendant et dans les conditions propres à permettre l'infection, d'une éventuelle activité infectieuse au sein de la culture-test.

Dans le premier type d'application envisagé ci-dessus, il peut n'être pas nécessaire de procéder à l'étape d'incubation terminale des lymphocytes T4X en l'absence de virus inactivé. Tel est en particulier le cas si, pour fixer le virus sur son récepteur cellulaire, on utilise un virus à la fois inactivé et marqué. Grâce au marquage, il devient alors possible de compter au sein d'une population initiale de lymphocytes T4, les proportions relatives de cellules T4X et de cellules T4Y que contenait la population initiale, au moyen de tout dispositif de détection approprié ou adapté au type de marquage utilisé. Un dispositif approprié consiste par exemple en un trieur de cellules si on veut séparer les T4X des T4Y.

Un mode préféré de réalisation de ce procédé consiste à utiliser du virus LAV marqué à la fluorescéine et un microscope à fluorescence.

Une variante du procédé consiste à utiliser du LAV couplé à une enzyme (peroxydase, β-galactosidase, phosphatase alcaline, etc.) par un agent bifonctionnel ne modifiant pas la reconnaissance du récepteur cellulaire par l'antigène viral (glutaraldéhyde ou la diaminobenzidine par exemple) et a révélé le complexe "LAV-T4X" par les techniques immunoenzymatiques bien connues telles que l'Elisa, mettant à profit la modification des propriétés d'absorption de radiations d'un substrat après hydrolyse de ce dernier par l'enzyme couplée au virus LAV.

Cette méthode a permis de constater de façon reproductible que les lymphocytes T4X représentent chez un sujet sain environ de 10 à 15 % de la population initiale des lymphocytes T4. Au contraire, et c'est là un résultat nouveau essentiel auquel l'invention a permis d'aboutir, l'expérience montre que la proportion relative des cellules T4X vis-à-vis des cellules T4Y est modifiée chez des personnes témoignant de signes cliniques caractéristiques des SLA ou du SIDA. Naturellement, on peut avoir recours à tout autre mode de séparation des lymphocytes T4X retenant le virus inactivé, par exemple lorsque celui-ci n'est pas lui-même marqué. Par exemple les lymphocytes sur lesquels le virus inactivé est fixé peuvent être détectés par des anticorps anti-LAV. La détection de ces anticorps peut être directe, notamment s'ils sont marqués par un marqueur fluorescent, radioactif ou enzymatique, ou indirecte, par exemple à l'aide d'anti-immunoglobulines marquées.

L'invention fournit donc une méthode de mesure in vitro du rapport lymphocytes T4X/T4Y à partir de tout échantillon biologique, notamment de sang provenant d'une personne soumise au diagnostic. La détermination d'un tel rapport permet, même chez des personnes porteuses d'anticorps anti-LAV, de s'assurer qu'en principe ces personnes porteuses d'anticorps ne sont pas menacées à brève échéance par la maladie, en d'autres termes ne sont pas atteintes de SLA ou de SIDA. Au contraire, si le rapport est modifié, on peut présumer de ce que la maladie est en train de se développer. Dans un tel cas, l'invention permet encore la détermination du stade de développement de la maladie et, si les mesures sont répétées dans le temps, de son évolution, notamment en corrélation avec les variations de ce rapport dans le sens d'une diminution.

Pour la première fois, l'invention fournit donc des moyens permettant la discrimination réelle souhaitée

8

entre les personnes asymptomatiques et les personnes déjà
malades. L'invention fournit donc encore de nouveaux paramètres efficacement substituables aux anciens, pour ce
qui est de la reconnaissance de l'installation de la maladie. Alors qu'antérieurement le rapport lymphocytes
T4/lymphocytes T8 ne pouvait guère être utilisé pour faire
ce dépistage, en raison de sa faible variation relative,
au moins jusqu'à un stade très avancé de la maladie, la
surveillance du rapport T4X/T4Y permet maintenant une surveillance étroite de l'évolution de la maladie, par conséquent le choix de la thérapeutique la plus efficace.

Un autre mode de mesure encore comprend par exemple l'étude de la variation des proportions des lymphocytes qui, au sein d'une population initiale de lymphocytes T4 mis en contact préalablement avec du virus inactivé, sont reconnus par les anticorps anti-T4. Ce test met à
profit la perturbation de la propriété de reconnaissance
que possèdent les anticorps anti-T4 pour les lymphocytes
T4, lorsque ces derniers ont auparavant été mis en présence de virus LAV. Il a en effet été constaté que, si
l'on incube une population de lymphocytes T4 en présence
d'anticorps anti-T4, ceux-ci tendent à se fixer sur toutes
les cellules T4. La fixation des anticorps anti-T4 est diminuée lorsque des lymphocytes T4 sont préalablement incubés en présence de virus LAV. On peut donc, par comptages
respectifs des lymphocytes T4X et des lymphocytes T4Y déterminer le rapport T4X/T4Y sus-indiqué. En variante, on
peut, après séparation des lymphocytes T4X, notamment dans
les conditions indiquées plus loin et internalisation du
virus inactivé dans les lymphocytes T4X, évaluer leur proportion relative par leur mise en contact avec une préparation de virus LAV inactivé et marqué, par exemple à la
fluorescéine, puis mesurer l'intensité de fluorescence
émise que l'on corrèle au nombre de lymphocytes T4X présents.

Ces tests soutiennent l'hypothèse (en même temps qu'il confirme ce qui a été montré plus haut, en ce qui concerne la proportion des lymphocytes T4X au sein d'une population de lymphocytes T4 chez un sujet sain) qu'il existe à la surface d'environ 10 % des lymphocytes T4 un récepteur qui reconnaît spécifiquement le LAV et que, lorsque ce dernier y est fixé, il masque le site de reconnaissance des lymphocytes T4 par les anticorps anti-T4. Cette constatation semble attribuable au fait que l'anticorps anti-T4 masque ce qui pourrait être le récepteur spécifique du LAV sur les lymphocytes T4X infectables, constatation qui trouve sa confirmation dans le fait que, à l'inverse, la fixation des anticorps anti-T4 sur les lymphocytes T4 empêche toute fixation du virus LAV sur les lymphocytes T4X.

De l'ensemble des constatations qui précèdent, il semble découler que les lymphocytes T4X "commandent" également les manifestations de certaines fonctions des lymphocytes T4Y, si l'on en juge par l'inhibition observée de l'expression du phénotype T4 et du récepteur pour l'IL-2 (appelé "TAC"), de l'ensemble des lymphocytes T4 chez des personnes déjà à un stade avancé du SIDA.

L'invention s'applique également à la séparation des lymphocytes T4X et T4Y, comme indiqué plus haut.

Un tel procédé est plus particulièrement intéressant à mettre en oeuvre sur des populations de cellules T4 appartenant à des lignées permanentes (CEM, H9, etc.) afin d'obtenir des cellules T4X permanentes toutes infectables par le virus LAV, de sorte que les rendements de production du virus peuvent être amplifiés.

Divers modes de réalisations de ce procédé peuvent être mis en oeuvre.

Un mode de réalisation préféré de ce procédé d'obtention de cellules T4X comprend la mise en contact d'une population de lymphocytes T4 avec un virus inactivé fixé

10

sur un support solide. Cette mise en contact est alors réalisée en milieu hétérogène constitué d'une phase fluide contenant la population initiale de cellules T4 et d'une phase solide retenant le virus inactivé. Les cellules T4X sont tout d'abord fixées sur le support solide par l'intermédiaire du virus, ce qui permet la séparation des deux phases. Après internalisation du virus inactivé, on observe alors un "décrochage" au moins partiel des lymphocytes T4X du support solide. Ces lymphocytes T4X peuvent alors être recueillis. Le support solide de ce procédé est de préférence constitué de billes de latex magnétiques ou de billes de polyacrylamide agarose du type de celles décrites dans la demande de brevet français n° 75 36889 publiée sous le n° 2 334 106 au nom de l'INSTITUT PASTEUR. Dans le cas de billes de latex magnétiques, la séparation est aisément réalisée à l'aide d'un aimant.

Dans un mode de réalisation du procédé selon l'invention, on réalise une incubation préalable de la population initiale de cellules T4 en présence de virus inactivé, puis on met en contact ces lymphocytes T4 avec des anticorps anti-T4, ou analogues, fixés sur un support insoluble adéquat. Les cellules T4Y, dont les récepteurs aux anticorps anti-T4 ne sont pas masqués par le virus LAV inactivé, se fixent par conséquent sur le support solide par l'intermédiaire des anticorps anti-T4, tandis que les cellules T4X fixées au virus LAV inactivé restent en suspension dans le fluide. Après séparation des deux phases, notamment par simple décantation et internalisation du virus inactivé par les cellules T4X, on obtient ainsi une population de cellules T4X, toutes infectables par le virus LAV. Cette population de cellules T4X présente encore la caractéristique qu'essentiellement la totalité de ses lymphocytes portent un récepteur pour le LAV virulent ou inactivé, récepteur qui paraît distinct des récepteurs pour les anticorps monoclonaux OKT4, puisque ceux-ci ne distinguent pas les lymphocytes T4X et T4Y entre eux.

L'invention concerne également un procédé de production du virus LAV par infection de populations essentiellement constituées de cellules T4X maintenues en culture et par récupération des virus produits par ces cellules.

Elle concerne aussi un procédé sensible de détection du caractère infectieux ou non d'une préparation biologique présumée contenir des antigènes de LAV ou HIV, par exemple d'un prélèvement d'un fluide biologique (par exemple de sérum) provenant d'un porteur d'anticorps anti-LAV, ce procédé consistant à mettre en contact ce prélèvement avec ladite population de T4X, à réaliser l'incubation du mélange pendant le temps et dans des conditions permettant l'infection éventuelle de ces lymphocytes T4X par un virus LAV ou HIV, et à détecter l'infection possible des lymphocytes T4X, par exemple grâce à la manifestation d'une activité reverse transcriptase en cas de réponse positive et, le cas échéant, la destruction complète à terme de cette population de T4X.

A l'inverse, comme déjà dit plus haut, ce procédé peut être mis en oeuvre dans des conditions semblables soit avec une préparation virale dont le caractère infectieux vis-à-vis de lymphocytes humains doit être déterminé, soit avec une préparation de rétrovirus dont le caractère infectieux pour l'homme avait déjà été reconnu, mais ce en présence de principes actifs de médicaments éventuels, dont doit être testée l'éventuelle capacité à inhiber l'action infectieuse de ce rétrovirus. Dans un cas particulier de cette dernière application, la préparation virale peut être celle qui avait été obtenue à partir d'un patient affecté (ou susceptible d'être affecté) par un SIDA, afin de déterminer celui des médicaments qui pourrait être le plus efficace en vue d'obtenir au moins une rémission du développement de la maladie chez le malade concerné.

Les techniques préférées de mise en oeuvre des procédés et méthodes et les résultats obtenus selon l'invention sont indiquées à titre d'exemples dans la description détaillée qui suit :

1) Marquage du virus LAV par de la fluorescéine.

1 ml de virus LAV (concentration protéique 5 mg/ml) purifié par ultracentrifugation sur gradient de saccharose 30-60 %, a été incubé 90 minutes à 25°C à l'obscurité en présence de 1 ml d'isothiocyanate de fluorescéine (FITC) à 2,5 mg/ml en tampon carbonate pH 9,2.

Le FITC non lié a ensuite été éliminé par gel filtration sur tamis moléculaire de Sephadex G25 de Pharmacia ou Biorad (qui retient l'isothiocyanate).

Le virus ainsi marqué est ensuite inactivé par un traitement à une température de 56°C ± 1°C.

2) Utilisation du virus LAV marqué à la fluorescéine pour étudier la population de lymphocytes T4X reconnue par le virus et pour séparer cette population à l'aide d'un trieur de cellules ou de toute autre méthode. Dans le cas d'un marquage du LAV par une enzyme, la séparation de cette population T4X se fait par utilisation d'un immuno-adsorbant.

Le marquage des cellules T4, préalablement fractionnées à l'aide d'anticorps monoclonaux spécifiques, est effectué en incubant $5.10^5$ de ces cellules avec 25 µl de LAV-FITC à 2,5 mg/ml pendant 60 minutes à 0 + 4°C. Après trois lavages par du PBS froid, les cellules sont alors observées à l'aide d'un microscope à fluorescence et utilisées pour le triage.

Le tableau I résume les résultats obtenus. On constate que dans une population de lymphocytes T4 préalablement fractionnés, seulement 10 % sont reconnus par FITC-LAV. Par contre, moins de 0,5 % d'une population de cellules T8 fractionnées sont marquées par ce virus. De même, on constate que des lymphocytes B et des monocytes ne sont pas ou sont peu reconnus par FITC-LAV, confirmant aussi que ces cellules présentent très peu de récepteurs pour le virus.

Dans les lignées continues (CEM ou $H_9$) utilisées pour produire le virus LAV en grandes quantités, on observe la présence de 10 à 28 % de cellules possédant le récepteur pour le virus constituant ainsi la population T4X.

A titre d'exemple, les lignées CEM clone 11 et CEM clone 13 ont été déposées à la Collection Nationale des Cultures de Micro-organismes de l'Institut Pasteur de Paris sous les numéros I-416 et I-417 respectivement.

### TABLEAU I
#### Marquage de différentes cellules par du LAV-FITC

| Cellules | % cellules fluorescentes * |
|---|---|
| $OKT_4^+$ | 10 |
| $OKT_8^+$ | 0,8 |
| Monocytes | 1 |
| B | 0,5 |
| CEM | 12 |
| CEM clone 11 | 10,4 |
| CEM clone 13 | 11 |
| $H_9$ | 28 |

* Moyenne d'au moins six expériences.

Afin de confirmer la spécificité d'un tel marquage, des expériences de blocage par différentes concentrations de virus froid ont été réalisées et les résultats sont résumés au tableau II. Pour ces expériences, des

0234973

14

cellules T4 ont été mises en contact avec des concentrations variables de virus purifié froid pendant une heure à 0 + 4°C, puis lavées et marquées par FITC-LAV dans les conditions préalablement décrites. Les résultats montrent qu'il existe une compétition pour la reconnaissance de la cellule cible entre le virus froid et le virus marqué, indiquant la spécificité de la fixation de FITC-LAV sur le récepteur par le virus à la surface de la cellule.

## TABLEAU II

### Expérience de blocage par du virus froid

| Concentration du virus LAV froid (mg/ml) | FITC-LAV (5 mg/ml) | % cellules T4 fluorescentes |
|---|---|---|
| 0 | + | 11 |
| 0,005 | + | 10 |
| 0,05 | + | 9,5 |
| 0,1 | + | 8,5 |
| 0,5 | + | 5,3 |
| 1 | + | 1,8 |
| 2,5 | + | 0,1 |
| 5 | + | 0,1 |

Enfin, avant d'envisager un triage des deux populations cellulaires, il a été vérifié qu'il était possible d'obtenir les mêmes résultats avec un virus FITC-LAV inactivé par chauffage pendant 30 minutes à 56°C. Les résultats indiqués dans le tableau III montrent que l'inactivation de FITC-LAV ne modifie en rien la spécificité du marquage. Par ailleurs, il a été également vérifié que les cellules T4X peuvent être infectées par une préparation virale virulente non fluorescente dès la première

15

heure après le marquage par FITC-LAV inactivé, ce dernier étant internalisé, suivant un processus d'endocytose, par les cellules T4X. Cette internalisation est accompagnée de la disparition de la fluorescence.

<u>TABLEAU III</u>

<u>Reconnaissance de la cellule cible T4 par FITC-LAV inactivée par la chaleur</u>

| FITC-LAV | % cellules fluorescentes | |
| --- | --- | --- |
| | cellules T4 | cellules T8 |
| non chauffé | 9 | O |
| chauffé 30 minutes 56°C | 16 | 1 |

3) Séparation des populations T4X et T4Y à l'aide de billes magnétiques.

La méthode précédente ne permettant pas d'obtenir une quantité importante de cellules, il a été développé une autre méthode de séparation utilisant des billes magnétiques de latex sur lesquelles une préparation de virus LAV purifié et inactivé par la chaleur a été fixée de manière covalente.

Le contact cellules-billes de latex est réalisé en incubant $10^7$ cellules T4 avec 200 µg de latex, ayant fixé le virus, pendant 15 minutes à 0 + 4°C dans une solution de PBS contenant 5 % de sérum de veau foetal, 1 % d'antibiotiques (mélange pénicilline, streptomycine, néomycine, commercialisé par Gibco) et 0,1 % d'azide de sodium. Puis à l'aide d'un aimant, on sépare les bille sur lesquelles sont fixées les cellules T4X du surnageant contenant la population T4Y.

Puis en marquant les deux populations cellulaires obtenues, à l'aide de FITC-LAV, il a ainsi été démontré que moins de 0,3 % de la population du surnageant T4Y pouvait être marquée par FITC-LAV et que la population T4X représentait environ 10 à 15 % de la population initiale de lymphocytes T4.

Par ailleurs, après infection de la population T4Y par une préparation virale virulente non marquée, il n'a pu être obtenu jusqu'à ce jour une réplication du virus LAV, ce qui suggère bien que seule la population T4X est infectable par le virus LAV.

4) Description du procédé de fixation des anticorps mono- clonaux sur boîte de Pétri et séparation des cellules T4X.

i) Préparation des plaques :

- les boîtes sont incubées à température ambiante pendant 1 heure avec 6 ml d'une solution contenant 10 µg/ml d'an- ticorps OKT4 en milieu Tris $510^{-2}$ mol/l pH 9,5, stérilisé par filtration ;

- on lave 4 fois en tampon PBS stérile ;

- on ajoute 5 ml par boîte de PBS contenant 1 % de sérum de veau foetal (FCS) et on incube pendant au moins 1 heure à température ambiante.

ii) Préparation des cellules :

$210^7$ lymphocytes T4 (T4) sont centrifugés 10 minutes à 450 g et à 4°C ;

le culot est repris dans 2 ml de PBS contenant 5 % FCS, pour obtenir un milieu contenant $10^7$ cellules/ml.

Les cellules sont incubées avec 0,75 mg de virus purifié, stérilisé par filtration et inactivé au préalable pendant 30 minutes à 56°C ; l'incubation a lieu à 4°C pendant 10 minutes (cellules et virus doivent être à 4°C au moment du contact) ; les cellules sont ensuite centrifugées 2 fois à 450 g pendant 10 minutes à 4°C, et le culot est repris dans 4 ml de PBS 5 % FCS.

iii) Séparation des cellules :

4 ml de milieu contenant $510^6$ cellules/ml sont

répartis dans la boîte ; on incube pendant 40 minutes à 4°C. La boîte est remuée doucement, puis on incube encore pendant 30 minutes à 4°C ; le surnageant est ensuite décanté doucement et la boîte lavée 4 fois avec du PBS contenant 1 % FCS doucement : les cellules ne s'étant pas fixées sur les anticorps OKT4 sont récupérées. Celles-ci forment alors la population de cellules T4X. On peut également récupérer les cellules T4Y, par exemple après lavage 4 fois en PBS contenant 1 % de FCS par des jets de seringue. Les cellules fixées sur l'OKT4 sont décrochées, ces cellules formant alors la population recherchée de lymphocytes T4Y.

REVENDICATIONS

1 - Population de cellules portant le phénotype T4 des lymphocytes T4, caractérisée en ce qu'elle est constituée essentiellement par des lymphocytes T4 possédant un récepteur du virus LAV, à l'état virulent ou inactivé, ce site étant susceptible d'être masqué par des anticorps anti-T4, cette population étant essentiellement exempte de lymphocytes T4 non infectables par le LAV et ne possédant pas de récepteur pour le virus LAV, à l'état virulent ou inactivé.

2 - Population de cellules selon la revendication 1, caractérisée en ce que ces cellules sont constituées par des lymphocytes T4 normaux.

3 - Population selon la revendication 1 caractérisée en ce que les cellules appartiennent à des lignées de cellules permanentes portant le phénotype T4 susdit.

4 - Procédé d'obtention de lymphocytes portant le phénotype T4 infectables par le virus LAV, essentiellement à l'exclusion de cellules portant le même phénotype mais non infectables par le LAV, caractérisé en ce que, partant d'une population de cellules portant ledit phénotype T4 et contenant à la fois des cellules infectables par le LAV (T4X) et des cellules à phénotype T4 non infectables par le LAV (T4Y), on met en contact cette population avec du virus LAV et l'on sépare les unes des autres les cellules qui ont fixé le virus LAV de celles qui ne l'ont pas fixé.

5 - Procédé selon la revendication 4, caractérisé en ce que le virus de préférence inactivé est fixé sur un support insoluble, et en ce que la population initiale de cellules en suspension dans un fluide est amenée au contact du virus inactivé fixé, et en ce que l'on sépare ensuite la phase solide retenant les lymphocytes T4X fixés de la phase fluide contenant les cellules T4Y non fixées et, après une incubation des lymphocytes T4X pendant un temps nécessaire à l'internalisation du virus inactivé, on récupère les lymphocytes T4X.

6 - Procédé selon la revendication 4, caractérisé en ce que le virus inactivé est à l'état de suspension dans un milieu fluide, en ce que l'on met en contact la population de cellules à séparer avec cette suspension de virus, et en ce que, pour opérer la séparation susdite, on met en contact la population de cellules avec des anticorps anti-T4 eux-mêmes fixés sur un support solide, et on récupère les cellules qui ne sont pas retenues par les anticorps anti-T4 fixés sur le support solide.

7 - Application du procédé selon l'une quelconque des revendications 1 à 6 à la détermination de la proportion de lymphocytes T4X au sein d'une population de lymphocytes T4 contenus dans un fluide biologique provenant d'une personne porteuse d'anticorps anti-LAV, en vue du diagnostic des SLA ou du SIDA chez ces personnes, un diagnostic positif étant corrélé à une diminution de la population T4X ou du rapport du nombre des lymphocytes T4X au nombre des lymphocytes T4Y, lesquels se comportent comme s'ils étaient dépourvus de récepteurs pour le virus LAV.

8 - Procédé de diagnostic _in_ _vitro_ des lymphadénopathies SLA ou du SIDA chez une personne dont le sérum contient des anticorps anti-LAV, ce procédé comprenant la mise en contact d'une population de lymphocytes T4 contenue dans un fluide biologique provenant de ladite personne, avec le virus LAV inactivé, la détection de la proportion des cellules retenant le virus inactivé et la détermination du rapport du nombre des lymphocytes (T4X) ayant fixé le virus au nombre des lymphocytes (T4Y) qui ne l'ont pas fixé, un diagnostic d'évolution de la maladie étant corrélé à une diminution de la proportion de lymphocytes T4X au sein de ladite population de lymphocytes T4 ou à une diminution du rapport T4X/T4Y vis-à-vis de la normale.

20

9 - Procédé selon la revendication 8, caractérisé en ce que le virus LAV inactivé est marqué et en ce que la mesure du rapport T4X/T4Y nécessaire au diagnostic _in vitro_ est appréciée par la proportion de marqueur retenue.

10 - Procédé selon la revendication 9, caractérisé en ce que le marqueur est immunofluorescent et en ce que l'on apprécie la valeur du rapport T4X/T4Y par l'intensité d'immunofluorescence du marqueur au sein de la population de lymphocytes T4X.

11 - Procédé selon la revendication 9, caractérisé en ce que le marqueur est immunoenzymatique et en ce que l'on apprécie la valeur du rapport T4X/T4Y par la modification des propriétés d'absorption des radiations d'un substrat lorsque ce dernier est hydrolysé par le marqueur.

12 - Procédé de production de virus LAV caractérisé par l'infection d'une population de lymphocytes T4X selon la revendication 3, de culture de cette population dans un milieu approprié et récupération du virus LAV produit.

Office européen
des brevets

**RAPPORT DE RECHERCHE EUROPEENNE**

Numéro de la demande
**0284973**

EP 87 40 0087

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| Y | SCIENCE, vol. 229, no. 4720, 27 septembre 1985, pages 1400-1402; J.A.HOXIE et al.: "Persistent noncytopathic infection of hormal human T lymphocytes with AIDS-associated retrovirus" * En entier * | 1-6 | C 12 N 5/00<br>C 12 Q 1/04<br>C 12 Q 1/70<br>G 01 N 33/569<br>C 12 N 7/00 //<br>G 01 N 33/543 |
| Y | THE JOURNAL OF IMMUNOLOGY, vol. 135, no. 5, novembre 1985, pages 3151-3162, The American Association of Immunologists; J.S.McDOUGAL et al.: "Cellular tropism of the human retrovirus HTLV-III/LAV. I. Role of T cell activation and expression of the T4 antigen" * En entier * | 1-6 | |
| A | NATURE, vol. 312, 20/27 décembre 1984, pages 763-767; A.G.DALGLEISH et al.: "The CD4 (T4) antigen is an essential component of the receptor for the AIDS retrovirus" * Page 765, paragraphe 4 - page 766, paragraphe 2 * | 1-6 | **DOMAINES TECHNIQUES RECHERCHES (Int. Cl.4)**<br><br>C 12 N<br>C 12 Q<br>G 01 N<br>C 12 D<br>A 61 K |

---   -/-

Le présent rapport de recherche a été établi pour toutes les revendications

| Lieu de la recherche | Date d'achèvement de la recherche | Examinateur |
|---|---|---|
| LA HAYE | 01-06-1987 | OSBORNE H.H. |

OEB Form 1503 03 82

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen
des brevets

**RAPPORT DE RECHERCHE EUROPEENNE**

**0234973**
Numéro de la demande

EP  87 40 0087

| Catégorie | Citation du document avec indication, en cas de besoin, des parties pertinentes | Revendication concernée | CLASSEMENT DE LA DEMANDE (Int Cl.4) |
|---|---|---|---|
| Y | NATURE, vol. 312, 20/27 décembre 1984, pages 767-768; D.KLATZMANN et al.: "T-lymphocyte T4 molecule behaves as the receptor for human retrovirus LAV" * En entier * | 1-6 | |
| Y | THE JOURNAL OF IMMUNOLOGY, vol. 136, no. 2, 16 janvier 1986, pages 361-363, The American Association of Immunologists; J.A.HOXIE et al.: "Infection of T4 lymphocytes by HTLV-III does not require expression of the OKT4 epitope" * Page 361, paragraphe 2 * | 1-6 | |
| D,Y | SCIENCE, vol. 225, 6 juillet 1984, pages 59-63; D.KLATZMANN et al.: "Selective tropism of lymphadenopathy associated virus (LAV) for helper-inducer T lymphocytes" * En entier * | 1-6 | DOMAINES TECHNIQUES RECHERCHES (Int. Cl.4) |

--- -/-

Le présent rapport de recherche a été établi pour toutes les revendications

| Lieu de la recherche | Date d'achèvement de la recherche | Examinateur |
|---|---|---|
| LA HAYE | 01-06-1987 | OSBORNE H.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen
des brevets

**RAPPORT DE RECHERCHE EUROPEENNE**

Numéro de la demande

**0234973**

EP  87 40 0087

| Catégorie | Citation du document avec indication, en cas de besoin, des parties pertinentes | Revendication concernée | CLASSEMENT DE LA DEMANDE (Int Cl 4) |
|---|---|---|---|
| | **DOCUMENTS CONSIDERES COMME PERTINENTS** | | Page 3 |
| Y | PROCEEDINGS OF THE NATIONAL ACADEMY OF SCIENCES, USA, vol. 82, août 1985, pages 5535-5539; L.S.KAMINSKY et al.: "High prevalence of antibodies to acquired immune deficiency syndrome (AIDS)-associated retrovirus (ARV) in AIDS and related conditions but not in other disease states" * En entier * | 1-6 | |
| A | IDEM | 7,12 | |
| D,P A | EP-A-0 176 429 (UNIVERSITE PIERRE & MARIE CURIE) * Abrégé; pages 8,9 * | 1-6 | |
| | | | **DOMAINES TECHNIQUES RECHERCHES (Int. Cl.4)** |

Le présent rapport de recherche a été établi pour toutes les revendications

| Lieu de la recherche | Date d'achèvement de la recherche | Examinateur |
|---|---|---|
| LA HAYE | 01-06-1987 | OSBORNE H.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82